# EUROPÄISCHE PATENTSCHRIFT

(11) **EP 0 576 976 B1**
(45) Veröffentlichungstag und Bekanntmachung des Hinweises auf die Patenterteilung: **13.09.1995**
(21) Anmeldenummer: 93109984.0
(22) Anmeldetag: 23.06.1993
(51) Int. Cl.: C07C 253/30, C07C 255/19, C07C 231/06

(54) **Verfahren zur Herstellung von 5-Cyanvaleriansäureamid**
Process for the preparation of 5-cyanovaleric amide
Procédé pour la préparation de l'amide 5-cyanovalérique

(30) Priorität: 01.07.1992 DE 4221604
(43) Veröffentlichungstag der Anmeldung: 05.01.1994
(73) Patentinhaber: BASF Aktiengesellschaft, 67063 Ludwigshafen (DE)
(72) Erfinder: Witzel, Tom, Dr., D-6700 Ludwigshafen (DE); Fuchs, Eberhard, Dr., D-6700 Ludwigshafen (DE); Merger, Franz, Dr., D-6710 Frankenthal (DE)

(56) Entgegenhaltungen:
- EP-A- 0 178 106
- DE-A- 2 320 060
- DE-A- 2 429 269
- US-A- 3 670 021
- JOURNAL OF ORGANIC CHEMISTRY. Bd. 15, Nr. 4 , Juli 1950 , EASTON US Seiten 800 - 1 R.H. WILEY ET. AL. 'Partial Hydrolysis of Adiponitrile and Sebaconitrile by Hydrogen Peroxide'
- CHEMISCHE BERICHTE Bd. 92, Nr. 10 , 1959 , WEINHEIM DE Seiten 2616 - 21 C. BERTHER 'Einseitige Verseifung von Dinitrilen und Hydrierung der entstandenen omega-Cyan-Säureamid'

## Beschreibung

Gegenstand der Erfindung ist ein Verfahren zur Herstellung von 5-Cyanvaleriansäureamid durch Umsetzung von Adipodinitril mit Wasser bei einer Temperatur von 50 bis 200°C in Gegenwart von nichtionisiertem Kupfer enthaltenden Trägerkatalysatoren.

Die Herstellung von 5-Cyanvaleriansäureamid aus Adipodinitril wurde schon mehrfach versucht. Aus J. org. Chem., Band 15 (1950) s. 800 bis 801 wird beschrieben, daß man 5-Cyanvaleriansäureamid durch Hydrolyse in Acetonlösung mit Wasserstoffperoxid in Gegenwart von Natronlauge erhält. Sowohl die Reaktionsführung als auch die Aufarbeitung sind sehr aufwendig, wobei zusätzlich nur eine Ausbeute von 30 % erzielt wird.

Nach einem anderen in Chem. Ber., Band 92 (1959) S. 2616 bis 2619 beschriebenen Verfahren zur Herstellung von 5-Cyanvaleriansäureamid wird Adipodinitril mit überschüssigem Wasser (Molverhältnis 1:50) an einem stark basischen Ionenaustauscher hydrolysiert. Dieses Verfahren hat den Nachteil, daß der Katalysator rasch desaktiviert wird durch Bildung von 5-Cyanvaleriansäure.

Weiter ist aus der DE-OS 24 29 269 bekannt, daß man Adipodinitril mit Wasser bei einer Temperatur von 100°C an einen Katalysator der Kupfer in eine Polymermatrix aus 4-Vinylpyridin und Divinylbenzol enthält, hydrolysiert. Das Verfahren hat den Nachteil, daß lange Reaktionszeiten erforderlich sind und 5-Cyanvaleriansäureamid nur mit 5 % Ausbeute anfällt, während das störende Adipinsäurediamid in einer Ausbeute von 10 % entsteht.

Es war deshalb die technische Aufgabe gestellt, ein Verfahren zur Herstellung von 5-Cyanvaleriansäureamid, ausgehend von Adipodinitril zur Verfügung zu stellen, das mit verbesserten Ausbeuten verläuft, wenig Adipinsäurediamid als Nebenprodukt anfällt, einfach durchführbar ist und der Katalysator eine hohe Lebensdauer hat und einfach regenerierbar ist.

Diese Aufgabe wird gelöst in einem Verfahren zur Herstellung von 5-Cyanvaleriansäureamid durch Umsetzen von Adipodinitril mit Wasser bei einer Temperatur von 50 bis 200°C in Gegenwart von nichtionisiertes Kupfer enthaltenden Trägerkatalysatoren, wobei man je Mol Adipodinitril 1 bis 15 Mol Wasser und eine Verweilzeit von 5 bis 60 Minuten anwendet.

Das neue Verfahren ist insofern bemerkenswert als im Hinblick auf die DE-OS 23 20 060 nicht zu erwarten war, daß man bei der Hydratisierung von Adipodinitril in Gegenwart von Kupfer enthaltenden Magnesiumsilikatkatalysatoren 5-Cyanvaleriansäureamid erhält, da ausweislich Beispiel 4 der genannten DE-OS ausschließlich Adipinsäurediamid erhalten wird.

Erfindungsgemäß setzt man Adipodinitril mit Wasser um, wobei man pro Mol Adipodinitril 1 bis 15 Mol Wasser, insbesondere 2 bis 11 Mol Wasser, anwendet.

Die Umsetzung wird bei einer Temperatur von 50 bis 200°C durchgeführt. Vorteilhaft hält man eine Temperatur von 70 bis 150°C ein. Ferner hält man in der Regel bei der Umsetzung einen Druck im Bereich von 100 bis 300.000 kPa, insbesondere 100 bis 50.000 kPa ein. Ferner hat es sich bewährt, wenn man darauf achtet, daß während der Umsetzung stets eine flüssige Phase eingehalten wird durch Abstimmung von Druck und Temperaturbedingungen.

Vorteilhaft führt man die Umsetzung unter Mitverwendung von in Wasser löslichen unter Reaktionsbedingungen inerten Lösungsmitteln durch. Geeignete Lösungsmittel sind beispielsweise Alkanole, insbesondere mit 1 bis 3 Kohlenstoffatomen wie Methanol, Ethanol oder Propanol, ferner Lactame wie Pyrrolidon und Caprolactam oder N-C₁-C₄-Alkyllactame wie N-Methylpyrrolidon, N-Methylcaprolactam oder N-Ethylcaprolactam. Weitere geeignete Lösungsmittel sind beispielsweise Tetrahydrofuran. In der Regel wendet man je Gewichtsteil Adipodinitril 1 bis 10 Gew.-Teile Lösungsmittel an.

Die Umsetzung wird erfindungsgemäß in Gegenwart von nichtionisiertes Kupfer enthaltenden Trägerkatalysatoren durchgeführt.

Als geeignete Trägermaterialien eignen sich beispielsweise Aluminiumoxid, Siliciumdioxid, deren Mischformen, Magnesium- und Aluminiumsilikate, Zirkondioxid und Titandioxid.

Eine besondere Ausführungsform betrifft nichtionisiertes Kupfer enthaltende Magnesiumsilikatkatalysatoren. Der Katalysator enthält im wesentlichen Maße nichtionisiertes Kupfer, das von vornherein z.B. in Gestalt von frisch reduziertem Kupfer dem Magnesiumsilikat vor der Behandlung mit reduzierenden Gasen zugeführt wird oder zweckmäßiger sich als Kupferverbindungen während der Behandlung bildet. Vorteilhaft wird das Kupfer aus den Kupferverbindungen stammen, die bei der Fällung des Magnesiumsilikats anwesend sind, und durch die reduktive Behandlung in die nichtionisierte Form übergehen. Im behandelten Katalysator können neben der Hauptmenge an nichtionisiertem Kupfer vorteilhaft von 0,1 bis 30, insbesondere 0,1 bis 10 Gew.-% des Gesamtkupfers noch als ein- und/oder zweiwertiges Kupfer, z.B. in Gestalt der ursprünglichen Kupferverbindung oder eine bei der Behandlung gebildeten Verbindung wie Kupfersilikat, vorliegen. Im allgemeinen kommen Mengen von 25 bis 70, insbesondere 30 bis 60 Gew.-% Gesamtkupfer, bezogen auf die Gewichtsmenge im Katalysator enthaltenen Magnesiumsilikat, in Betracht.

Als Kupferverbindungen sind z.B. das -nitrat, -sulfat, -chlorid, -oxid, -hydroxid, -tartrat, -acetat des einwertigen oder zweckmäßig des zweiwertigen Kupfers geeignet. Das Magnesiumsilikat wird in Gegenwart einer zweckmäßig das nichtionisierte Kupfer liefernden Kupferverbindung durch Fällung einer Magnesiumverbindung mit einem Alkalimetallsilikat, zweckmäßig ein Kalium- oder Natriumsilikat, in der Regel in einem wäßrigen Fällungsmedium, vorteilhaft in einem Verhältnis von 1 bis 5 Mol Alkalimetallsilikat je Mol Magnesiumverbindung hergestellt. Als Magnesiumverbindungen kommen in Frage: Magnesiumnitrat, -sulfat, -chlorid, -tartrat, -acetat, -oxalat. Die Fällung wird vorteilhaft bei einer Temperatur von 15 bis 50°C durchgeführt. Die Kupferverbindung kann im Fällungsmedium teilweise suspendiert sein.

Der Katalysator kann kleine Mengen an Zink, Cadmium, Chrom, Molybdän, Wolfram, Vanadin, Titan und/oder Thorium in Gestalt der Metalle oder zweckmäßig entsprechende Verbindungen enthalten. Vorteilhaft kommen Mengen von 1 bis 30, insbesondere 1 bis 10 Gew.-% dieser Zusatzmetalle, bezogen auf die Gewichtsmenge des im Katalysator enthaltenen Magnesiums, in Frage.

Geeignete Katalysatoren erhält man beispielsweise durch Fällung einer Magnesiumverbindung in Gegenwart von Kupferverbindungen gegebenenfalls einer oder mehrerer Verbindungen der Zusatzmetalle in wäßrigem Medium nachdem in dem deutschen Reichspatent 869 052 beschriebenen Verfahren. Zweckmäßig wird die gefällte Masse nach dem Waschen und Trocknen mit einer Alkalimetallsilikatlösung z.B. einer 5 bis 20 gew.-%igen Natrium- oder Silikatlösung geknetet und nach dem abermaligen Trocknen bei schwach erhöhter Temperatur, z.B. bis zu 30°C, verformt. Vorteilhaft werden 10 bis 50 gew.-^{%}ige wäßrige Lösungen der genannten Kupfer- und Magnesiumverbindungen und 10 bis 30 gew.-%ige wäßrige Lösungen von Alkalimetallsilikat miteinander bei vorgenannter Fällungstemperatur gemischt und während 1 bis 60 Minuten die Fällung durchgeführt. Dann wird der Niederschlag zweckmäßig abgesaugt, mit Wasser gewaschen, z.B. bis zur Entfernung des ursprünglichen Kupferanions, wie des Nitratanions, dann bei 20 bis 30°C vorgetrocknet, verformt, z.B. in Tabletten, Kugeln oder Strängen, und dann bei 50 bis 70°C getrocknet.

Der so erhaltene Katalysator wird in der Regel bei erhöhter Temperatur z.B. bei 100 bis 230°C, vorzugsweise bei 180 bis 230°C, drucklos oder unter Druck kontinuierlich oder diskontinuierlich mit reduzierenden Gasen behandelt. In der Regel führt man die Reduktion mit Wasserstoff durch. Die Reduktionszeit beträgt zweckmäßig 1 bis 15 Stunden. Vorteilhaft wird der Katalysator nach der Herstellung und Trocknung zuerst unter Stickstoff, vorzugsweise in einem Stickstoffstrom, z.B. bei 100 bis 180°C, zweckmäßig 0,5 bis 3 Stunden erhitzt. Dann erfolgt die reduktive Behandlung, vorzugsweise über eine Dauer von 0,5 bis 24 Stunden. Wobei man im allgemeinen 5 bis 15 Mol Wasserstoff je Kilogramm Katalysator anwendet. Die Herstellung geeigneter Katalysatoren wird beispielsweise beschrieben in der DE-A 27 51 336, DE-OS 23 20 060 und der DE-OS 23 20 061.

Eine weitere bevorzugte Ausführungsform betrifft nichtionisiertes Kupfer enthaltende Trägerkatalysatoren aus Aluminiumoxid. Solche Katalysatoren sind in der EP-A 44 444 ausführlich beschrieben, so daß sich nähere Ausführungen hierüber erübrigen. Besonders bevorzugt führt man die reduktive Behandlung dieser Katalysatoren analog zu dem in der DE-A 27 51 336 ausführlich beschriebenen Verfahren durch.

Des weiteren ist es erfindungsgemäß, daß man bei der Umsetzung eine Verweilzeit von 5 bis 60 Minuten einhält. Bei einer Verweilzeit unter 5 Minuten erhält man im allgemeinen einen zu geringen Umsatz, bei einer Verweilzeit von mehr als 60 Minuten erhält man in der Regel eine zu niedrige Selektivität.

Die Umsetzung läßt sich absatzweise oder vorzugsweise kontinuierlich durchführen. Vorteilhaft leitet man das Reaktionsgemisch in Sumpfphase in einem Rohr über einen fest angeordneten Katalysator und hält die angegebenen Reaktionsbedingungen ein. Aus dem erhaltenen Reaktionsgemisch läßt sich 5-Cyanvaleriansäuremonoamid unschwer durch Extraktion oder insbesondere Destillation gewinnen.

Das neue Verfahren hat den Vorteil, daß die Bildung von Adipinsäurediamid minimiert wird. Das Verfahren hat zudem den Vorteil, daß die verwendeten Katalysatoren eine lange Lebensdauer haben und leicht regenerierbar sind. Ferner hat das neue Verfahren den Vorteil, daß es kontinuierlich durchführbar ist und Ausbeuten liefert, die eine technische Realisierung ermöglichen. 5-Cyanvaleriansäureamid ist ein wichtiges Vorprodukt für Caprolactam.

Das Verfahren nach der Erfindung sei an folgenden Beispielen veranschaulicht.

### Beispiel 1

In einem Edelstahlreaktor (Länge 1000 mm; Durchmesser 10 mm) wurde ein Katalysator (60 g als 1.5 mm Stränge) fest angeordnet, der 30 Gew.-% Kupfer in Gegenwart von Magnesiumsilikaten enthielt und dessen Zusammensetzung in der DE 27 51 336 angegeben ist. Die reduktive Behandlung des Katalysators, die ebenfalls in der DE 27 51 336 beschrieben ist, wurde direkt im Reaktor durchgeführt. Nach der Reduktion leitete man je Stunde von unten nach oben Adipodinitril und Wasser.

Die Reaktionsparameter sind in Tabelle 1 aufgelistet.

**Tabelle 1**

| | Temp. [°C] | ADN/H₂O [mol/mol] | ADN/Kat. [kg/kg*h] | VWZ [min] | Sel. [%] | Ausb. [%] | Ums. [%] |
|---|---|---|---|---|---|---|---|
| a) | 100 | 1/10 | 7 | 5 | 72 | 19 | 26 |
| b) | 100 | 1/5 | 11 | 5 | 74 | 10 | 13 |
| c) | 100 | 1/1 | 20 | 5 | 100 | 1 | 1 |
| ADN = Adipodinitril; Sel. = Selektivität VWZ = Verweilzeit; Aus. = Ausbeute; Ums. = Umsatz | | | | | | | |

Nach Einengen und Destillation eines Reaktionsaustrags (Beispiel 1a) erhielt man aus 100 g Produktaustrag: 70 g Adipodinitril, 15 g 5-Cyanvaleriansäureamid (Schmp.: 63 bis 65°C; Sdp. _{0,5}: 148 bis 167°C) sowie einen Rückstand von Adipinsäurediamid (5 g; Schmp.: 224 bis 227°C).

### Beispiel 2

In einem Glaskolben wurden 1 mol Adipodinitril und 5 mol Wasser in 80 g Ethanol gelöst und mit 20 g eines Katalysators, der 40 Gew.-% Kupfer in Gegenwart von Aluminiumoxid enthielt, und dessen Zusammensetzung in der EP-A 44 444 (Beispiel 1) angegeben ist, versetzt. Die reduktive Behandlung des Katalysators wurde, wie in der EP-A 44 444 beschrieben, bei 100 kPa in einem Glasreaktor durchgeführt.

Die Suspension erhitzte man für 45 min. auf 90°C. Danach hatten sich 7 % Adipodinitril zu 5-Cyanvaleriansäureamid mit einer Selektivität von 88 % umgesetzt.

## Patentansprüche

1. Verfahren zur Herstellung von 5-Cyanvaleriansäureamid durch Umsetzung von Adipodinitril mit Wasser bei einer Temperatur von 50 bis 200°C in Gegenwart von nichtionisiertes Kupfer enthaltenden Trägerkatalysatoren, dadurch gekennzeichnet, daß man je Mol Adipodinitril 1 bis 15 Mol Wasser und eine Verweilzeit von 5 bis 60 Minuten anwendet.

2. Verfahren nach Anspruch 1, dadurch gekennzeichnet, daß man eine Temperatur von 70 bis 150°C einhält.

3. Verfahren nach den Ansprüchen 1 und 2, dadurch gekennzeichnet, daß man je Mol Adipodinitril 2 bis 11 Mol Wasser anwendet.

4. Verfahren gemäß den Ansprüchen 1 bis 3, dadurch gekennzeichnet, daß man einen Kupfer enthaltenden Trägerkatalysator, ausgewählt aus der Gruppe, bestehend aus Aluminium- und Magnesiumsilikat, Aluminiumoxid, Siliciumdioxid, Titandioxid und Zirkondioxid, verwendet.

5. Verfahren gemäß den Ansprüchen 1 bis 4, dadurch gekennzeichnet, daß der Katalysator 1 bis 30 Gew.-% mindestens eines Elements oder einer Verbindung, ausgewählt aus der Gruppe, bestehend aus Zink, Cadmium, Chrom, Molybdän, Wolfram, Vanadin, Titan und Thorium, enthält.

6. Verfahren gemäß den Ansprüchen 1 bis 5, dadurch gekennzeichnet, daß man mit Wasser mischbare unter Reaktionsbedingungen inerte Lösungsmittel mitverwendet.

7. Verfahren gemäß den Ansprüchen 1 bis 6, dadurch gekennzeichnet, daß man Alkanole mit 1 bis 3 Kohlenstoffatomen als Lösungsmittel mitverwendet.

## Claims

1. A process for preparing 5-cyanovaleramide by reacting adiponitrile with water at from 50 to 200°C in the presence of a supported unionized-copper catalyst, which comprises employing from 1 to 15 mol of water per mole of adiponitrile and a residence time of from 5 to 60 minutes.

2. A process as claimed in claim 1, wherein a temperature of from 70 to 150°C is maintained.

3. A process as claimed in claim 1 or 2, wherein from 2 to 11 mol of water are employed per mole of adiponitrile.

4. A process as claimed in any of claims 1 to 3, wherein the supported copper catalyst used is selected from the group consisting of aluminum silicate, magnesium silicate, aluminum oxide, silicon dioxide, titanium dioxide and zirconium dioxide.

5. A process as claimed in any of claims 1 to 4, wherein the catalyst contains from 1 to 30% by weight of at least one element or compound selected from the group consisting of zinc, cadmium, chromium, molybdenum, tungsten, vanadium, titanium and thorium.

6. A process as claimed in any of claims 1 to 5, wherein a water-miscible solvent that is inert under reaction conditions is present.

7. A process as claimed in any of claims 1 to 6, wherein an alkanol of from 1 to 3 carbon atoms is present as solvent.

## Revendications

1. Procédé de préparation de 5-cyanovaléramide par réaction d'adipodinitrile avec de l'eau à une température de 50 à 200°C en présence de catalyseurs sur support contenant du cuivre non ionisé, caractérisé en ce que l'on utilise 1 à 15 moles d'eau par mole d'adipodinitrile et on applique une durée de séjour de 5 à 60 min.

2. Procédé selon la revendication 1, caractérisé en ce que l'on maintient une température de 70 à 150°C.

3. Procédé selon la revendication 1 ou 2, caractérisé en ce que l'on utilise 2 à 11 moles d'eau par mole d'adipodinitrile.

4. Procédé selon l'une quelconque des revendications 1 à 3, caractérisé en ce que l'on utilise un catalyseur sur support contenant du cuivre qui est choisi dans le groupe constitué par le silicate d'aluminium et de magnésium, l'oxyde d'aluminium, le dioxyde de silicium, le dioxyde de titane et le dioxyde de zirconium.

5. Procédé selon l'une quelconque des revendications 1 à 4, caractérisé en ce que le catalyseur contient de 1 à 30% en poids d'au moins un élément ou un composé choisi dans le groupe constitué par le zinc, le cadmium, le chrome, le molybdène, le tungstène, le vanadium, le titane et le thorium.

6. Procédé selon l'une quelconque des revendications 1 à 5, caractérisé en ce que l'on utilise simultanément des solvants miscibles à l'eau, inertes dans les conditions de la réaction.

7. Procédé selon l'une quelconque des revendications 1 à 6, caractérisé en ce que l'on utilise simultanément, comme solvants, des alcanols à 1-3 atomes de carbone.
